# EUROPEAN PATENT SPECIFICATION

(11) **EP 3 856 230 B1**
(45) Date of publication and mention of the grant of the patent: **03.09.2025**
(21) Application number: 19865910.4
(22) Date of filing: 26.09.2019
(51) Int. Cl.: A61K 38/22

(54) **STORAGE SOLUTION COMPOSITIONS FOR THE STABILIZATION OF PURIFIED MÜLLERIAN INHIBITING SUBSTANCE (MIS) AND METHODS OF USE AND PRODUCTION RELATED THERETO**
LAGERLÖSUNGSZUSAMMENSETZUNGEN ZUR STABILISIERUNG VON GEREINIGTER MÜLLER-HEMMUNGSSUBSTANZ (MIS) UND VERFAHREN ZUR VERWENDUNG UND HERSTELLUNG IN ZUSAMMENHANG DAMIT
COMPOSITIONS DE SOLUTION DE STOCKAGE POUR LA STABILISATION D'UNE SUBSTANCE INHIBITRICE MÜLLÉRIENNE PURIFIÉE ET PROCÉDÉS D'UTILISATION ET DE PRODUCTION ASSOCIÉS

(30) Priority: 28.09.2018 US 201862739105 P
(43) Date of publication of application: 04.08.2021
(73) Proprietor: Siemens Healthcare Diagnostics Inc., Tarrytown, NY 10591 (US)
(72) Inventor: CHUANG, Tom, Elmsford New York 10523 (US); BOGDANOVIC, Jelena, Elmsford New York 10523 (US); SINHA, Seema, Rancho Santa Margarita California 92688 (US); HAN, Aili, Cypress California 90630 (US); JACEWICZ, Agata, New York New York 10044 (US); TRAN, Tramanh, Yonkers New York 10701 (US)
(74) Representative: Schweitzer, Klaus
(86) International application number: PCT/US2019/053062
(87) International publication number: WO 2020/069064

(56) References cited:
- US-A- 5 328 844
- US-A- 5 661 126
- US-A- 6 165 981
- US-A- 6 165 981
- US-A1- 2007 161 015
- US-A1- 2018 236 034
- PEPINSKY R B ET AL: "PROTEOLYTIC PROCESSING OF MULLERIAN INHIBITING SUBSTANCE PRODUCES ATRANSFORMING GROWTH FACTOR-BETA-LIKE FRAGMENT", JOURNAL OF BIOLOGICAL CHEMISTRY, AMERICAN SOCIETY FOR BIOCHEMISTRY AND MOLECULAR BIOLOGY, US, vol. 263, no. 35, 15 December 1988 (1988-12-15), pages 18961 - 18964, XP002024938, ISSN: 0021-9258
- TENG ET AL: "Quantification of Mullerian inhibiting substance in developing chick gonads by a competitive enzyme-linked immunosorbent assay", DEVELOPMENTAL BIOLOGY, ELSEVIER, AMSTERDAM, NL, vol. 123, no. 1, 1 September 1987 (1987-09-01), pages 255 - 263, XP024787578, ISSN: 0012-1606, [retrieved on 19870901], DOI: 10.1016/0012-1606(87)90447-7
- ROCHE: "Micr-O-protect Highly efficient non-toxic biocide mixture with broad specificity", ROCHE, May 2004 (2004-05-01), XP055699142, Retrieved from the Internet <URL:https://isg.ku.edu.tr/sites/isg.ku.edu.tr/files/laboratuvar/med/msds/MicrOProtect11585720001.pdf> [retrieved on 20191118]
- ANONYMOUS: "Chloramphenico)", WIKIPEDIA., 26 March 2018 (2018-03-26), XP055699140, Retrieved from the Internet <URL:https://en.wikipedia.org/w/index.php?title=Chloramphenico)&lidid=832553530> [retrieved on 20191118]
- ANONYMOUS: "Polysorbate 20", WIKIPEDIA., 11 April 2016 (2016-04-11), XP055699138, Retrieved from the Internet <URL:https://en.wikipedia.org/w/index.php?title=Polysorbate_20&oldid=714786804> [retrieved on 20191118]

## Description

### CROSS REFERENCE TO RELATED APPLICATIONS

Not Applicable.

### STATEMENT REGARDING FEDERALLY FUNDED RESEARCH OR DEVELOPMENT

Not Applicable.

### TECHNICAL FIELD

The presently disclosed and claimed inventive concept(s) relate to a composition(s), kit(s), and method(s) that increase the stability and/or shelf life of component(s) and/or reagent(s) utilized for the conductance of at least one diagnostic assay. More specifically, the presently disclosed and claimed inventive concept(s) relate to non-limiting embodiments of a storage buffer solution that stabilizes and increases the shelf-life of purified Müllerian Inhibiting Substance (MIS), as well as kits and methods of use and production related thereto. US-6165981 discloses on page 6 lines 5-16 a Müllerian Inhibiting Substance storage buffer composition, comprising: deionized water; at least one buffering agent ( In the document sodium phosphate, HEPPSO, col 4 line 39); at least one salt (NaCl); at least one sugar alcohol (trehalose); gelatin (gelatin); at least one surfactant (EDTA); protease-free bovine serum albumin (BSA); and at least one biocide (preservative). 2 The difference is the alcohol sugar. In the document is a sugar used. The document discloses the use of detergent (surfactant) on col. 5 line 46-52 . XP-002024938 discloses MIS storage buffer ( 10% glucose, 300 mM NaCl, 10 mM HEPES, pH 7.5)

### BACKGROUND

Müllerian Inhibiting Substance (MIS)/Anti-Müllerian Hormone (AMH) is a labile dimeric glycoprotein and, due to its carboxy-terminal amino acid homology, MIS is a member of the transforming growth factor-beta (TGF-β) superfamily of glycoproteins that are involved in the regulation of growth and differentiation, including, for instance, inhibition of oocyte meiosis and lung surfactant. MIS is a gonadal hormone responsible for the regression of the paramesonephric/Müllerian ducts, the anlagen of the female reproductive tract in the fetal urogenital ridge, during male embryogenesis. MIS has also been utilized as a clinical and/or diagnostic reagent and/or biomarker for certain biological diseases and/or conditions, including, without limitation, general fertility assessments, predictive likelihood of success for *in-vitro* fertilization procedures, diagnosis of certain cancers (such as, by way of example, ovarian cancer and uterine cancer), detection of polycystic ovary syndrome, contraception, and/or treatment of endometriosis and/or adenomyosis.

As is the case with many TGF-β proteins, MIS is produced as a dimeric precursor and undergoes post-translational processing for activation, requiring cleavage and dissociation to release bioactive C-terminal fragments. Once purified via methods commonly known in the art, including, without limitation, affinity purification and/or ion-exchange methodologies, the purified MIS is highly prone and susceptible to cleavage and degradation by proteolytic enzymes, thereby resulting in the decreased shelf-life and a loss of bioactivity of the purified MIS.

Accordingly, there is a need for improved compositions, such as, by way of example only, improved storage buffers, and methods that increase the shelf-life and stabilize and substantially mitigate the loss of bioactivity of purified MIS. It is to such compositions and methods, as well as kits related thereto, that the presently disclosed and claimed inventive concept(s) is directed.

### DESCRIPTION OF THE SEVERAL VIEWS OF THE DRAWINGS

Figures 1A-1E are graphical plots showing the bioactivity of MIS over a sixty (60)-day period for initial concentrations of MIS (at day 0) ranging from about 0.15 ng/mL to about 33.50 ng/mL in accordance with the presently disclosed and/or claimed inventive concept(s).
Figure 2 is a box diagram of a non-limiting embodiment a process for the extraction, purification, storage, and stabilization of purified MIS in a storage buffer constructed in accordance with the presently disclosed and/or claimed inventive concept(s).

### DETAILED DESCRIPTION

The invention is limited to the scope of the appended claims.

Unless otherwise defined herein, scientific and technical terms used in connection with the presently disclosed and claimed inventive concept(s) shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art.

All patents, published patent applications, and non-patent publications mentioned in the specification are indicative of the level of skill of those skilled in the art to which this presently disclosed and claimed inventive concept(s) pertains.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The singular forms "a," "an," and "the" include plural referents unless the context clearly indicates otherwise. Thus, for example, reference to "a compound" may refer to 1 or more, 2 or more, 3 or more, 4 or more or greater numbers of compounds. The term "plurality" refers to "two or more." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects. For example but not by way of limitation, when the term "about" is utilized, the designated value may vary by ± 20% or ± 10%, or ± 5%, or ± 1%, or ± 0.1% from the specified value, as such variations are appropriate to perform the disclosed methods and as understood by persons having ordinary skill in the art. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z. The use of ordinal number terminology (i.e., "first", "second", "third", "fourth", etc.) is solely for the purpose of differentiating between two or more items and is not meant to imply any sequence or order or importance to one item over another or any order of addition, for example.

As used in this specification and claim(s), the terms "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, the term "substantially" means that the subsequently described event or circumstance occurs at least 90% of the time, or at least 95% of the time, or at least 98% of the time.

As used herein, the phrase "associated with" includes both direct association of two moieties to one another as well as indirect association of two moieties to one another. Non-limiting examples of associations include covalent binding of one moiety to another moiety either by a direct bond or through a spacer group, non-covalent binding of one moiety to another moiety either directly or by means of specific binding pair members bound to the moieties, incorporation of one moiety into another moiety such as by dissolving one moiety in another moiety or by synthesis, and coating one moiety on another moiety.

The term "patient" includes human and veterinary subjects. In certain embodiments, a patient is a mammal. In certain other embodiments, the patient is a human. "Mammal" for purposes of treatment refers to any animal classified as a mammal, including human, domestic and farm animals, nonhuman primates, and zoo, sports, or pet animals, such as dogs, horses, cats, cows, etc.

Turning now to particular embodiments, the presently disclosed and claimed inventive concept(s) relate to composition(s) comprising and/or consisting of an MIS storage buffer, as well as method(s) of stabilization MIS related thereto as disclosed in the appended claims.

It is contemplated that virtually any reagent used in the fields of biological, chemical, or biochemical analyses and assays could be used in the devices, kits, and methods of the presently claimed and disclosed inventive concept(s). It is contemplated that these reagents may undergo physical and/or chemical changes when bound to an analyte of interest whereby the intensity, nature, frequency, or type of signal generated by the reagent-analyte complex is directly proportional or inversely proportional to the concentration of the analyte existing within the fluid sample. These reagents may contain indicator dyes, metal, enzymes, polymers, antibodies, and electrochemically reactive ingredients and/or chemicals that, when reacting with an analyte(s) of interest, may exhibit change in color.

Any method of detecting and measuring an analyte in can be used in the devices, kits, and methods of the presently claimed and inventive concepts. A variety of assays for detecting analytes are well known in the art and include, but are not limited to, chemical assays, enzyme inhibition assays, antibody stains, latex agglutination, latex agglutination inhibition and immunoassays, such as, radioimmunoassays. The term "antibody" herein is used in the broadest sense and refers to, for example, intact monoclonal antibodies, polyclonal antibodies, multi-specific antibodies (e.g., bispecific antibodies), and to antibody fragments that exhibit the desired biological activity (e.g., antigen/analyte-binding). The antibody can be of any type or class (e.g., IgG, IgE, IgM, IgD, and IgA) or sub-class (e.g., IgG1, IgG2, IgG3, IgG4, IgA1, and IgA2).

Assays, including, but not limited to, immunoassays, nucleic acid capture assays, lipid-based assays, and serology-based assays, can be developed for a multiplexed panel of proteins, peptides, and nucleic acids which may be contained within a liquid test sample, with such proteins and peptides including, for example but not by way of limitation, albumin, microalbumin, cholesterol, triglycerides, high-density lipoproteins, low-density lipoproteins, hemoglobin, myoglobin, α-1-microglobin, immunoglobins, enzymes, proteins, glycoproteins, protease inhibitors, drugs, cytokines, creatinine, and glucose.

Any method of biological and/or chemical purification can be used in the presently disclosed and/or claimed inventive concept(s). For instance, by way of example only, when the compound to be purified is at least one protein, the at least one protein may be purified, after extraction, via size exclusion chromatography, hydrophobic interaction chromatography (HIC), ion exchange chromatography, free-flow electrophoresis, affinity chromatography, immunoaffinity chromatography, metal binding techniques, high performance liquid chromatography (HPLC), and combinations thereof.

In one non-limiting embodiment, the presently disclosed and/or claimed inventive concept(s) is directed to a new and improved storage buffer composition that preserves the bioactivity of MIS as disclosed in the claims.

In one non-limiting embodiment, the improved storage buffer comprises deionized water;
at least one buffering agent;
at least one salt;
at least one sugar alcohol, wherein the at least one sugar alcohol is mannitol;
gelatin;
at least one surfactant;
protease-free bovine serum albumin;
and at least one biocide,
wherein the Müllerian Inhibiting Substance storage buffer composition is suitable to be stored for use at a temperature range from about 2°C to about 8°C.

The at least one buffering agent may comprise, consist of, or be selected from the group consisting of 4-(2-hydroxyethyl)-1-piperazineethanesulfonic acid (HEPES) buffer, [tris(hydroxymethyl)methylamino]propanesulfonic acid (TAPS) buffer, 2-(bis(2-hydroxyethyl)amino)acetic acid (bicine) buffer, tris(hydroxymethyl)aminomethane (TRIS) buffer, 3-[N-tris(hydroxymethyl)methylamino]-2-hydroxypropanesulfonic acid (tricine) buffer, 2-[[1,3-dihydroxy-2-(hydroxymethyl)propan-2-yl]amino]ethanesulfonic acid (TES) buffer, 3-(N-morpholino)propanesulfonic acid (MOPS) buffer, piperazine-N-N'-bis(2-ethanesulfonic acid) (PIPES) buffer, dimethylarsenic acid (cacodylate), 2-(N-morpholino)ethanesulfonic acid (MES) buffer, N-cyclohexyl-2-aminoethanesulfonic acid (CHES) buffer, citric acid buffer, acetic acid buffer, monopotassium phosphate buffer, borate buffer, and combinations thereof. In one non-limiting embodiment of the presently disclosed inventive concept(s), the at least one buffering agent is HEPES buffer having a concentration of about 50 mM.

The at least one salt may be any salt commonly known in the art, including, without limitation, any salt(s) that are formed from the chemical reaction of a base and an acid, a metal and an acid, a metal and a non-metal, a base and an acid anhydride, an acid and a base anhydride, the solubilization and recombination of two or salts, and combinations thereof. In one non-limiting embodiment of the presently disclosed inventive concept(s), the at least one salt is sodium chloride having a concentration of about 150 mM.

In one non-limiting embodiment of the presently disclosed and/or claimed inventive concept(s), the gelatin is a purified, liquid gelatin, for instance, by way of example only, HiPure Liquid Gelatin commercially offered for sale by Norland Products, Inc. having a concentration of about 6 grams/liter.

The at least one sugar alcohol is mannitol having a concentration of about 50 grams/liter.

The at least one surfactant may comprise, consist of, or be selected from the group consisting of an anionic surfactant(s), non-ionic surfactant(s), cationic surfactant(s), amphoteric surfactant(s), zwitterionic surfactant(s), and combinations thereof. In one non-limiting embodiment of the presently disclosed and/or claimed inventive concept(s), the at least one surfactant is polyethylene glycol sorbitan monolaurate (commercially sold by Millipore Sigma as Tween^{®} 20) having a concentration of about 1.1 grams/liter.

The at least one biocide may comprise, consist of, or be selected from the group consisting of any substance or combination of substances, including, without limitation, preservatives, antimicrobial agents (including, but not limited to, germicides, antibiotics, antibacterials (including, bactericides), antivirals, antifungals, antiprotozoals, and/or antiparasites), anti-fouling agents, disinfectants, and/or pesticides (including, but not limited to, fungicides, herbicides, insecticides, algicides, molluscicides, miticides, and/or rodenticides) which are used for the control of organisms that are harmful to human and/or animal health and/or that cause damage to natural or manufactured products. Biocides, as used herein, can be of any form, including, without limitation, aqueous (i.e., a fluid) or solid (i.e., a powder). In one non-limiting embodiment of the presently disclosed and/or claimed inventive concept(s), the at least one biocide is an ethanolic solution of brom-nitro-dioxane and methylisothiazolone (MIT) (commercially sold by Roche Diagnostics as Micr-O-protect) having a concentration of about 2 milliliters/liter.

A non-limiting embodiment of an improved MIS storage buffer composition constructed in accordance with the presently disclosed and/or claimed inventive concept(s) is shown hereinbelow in Table 1.

**Table 1: Non-Limiting Embodiment of MIS Storage Buffer Composition**

| **Ingredient** | **Concentration** | **Additional Concentration Info.** |
|---|---|---|
| D.I. Water | | |
| HEPES (MW = 238.30) | 6.60 g/L | 50 mM HEPES buffer |
| HEPES Sodium Salt (MW = 260.29) | 5.78 g/L | |
| Sodium Chloride (MW = 58.44) | 8.77 g/L | 150 mM |
| D- Mannitol | 50 g/L | |
| Liquid Gelatin | 6 g/L | |
| Tween-20 (surfactant) | 1.1 g/L | |
| BSA, protease free (≥98%) | 30 g/L | |
| Micr-O-protect Biocide | 2 mL/L | |

Once composed, the pH of the MIS storage buffer is adjusted to be neutral (about 7.5). In addition, the storage buffer may be filtered (for instance, by way of example only, through a 0.2 micrometer filter) to filter out any particulate matter from the storage buffer. The buffer is then stored for use at a temperature ranging from about 2°C to about 8°C.

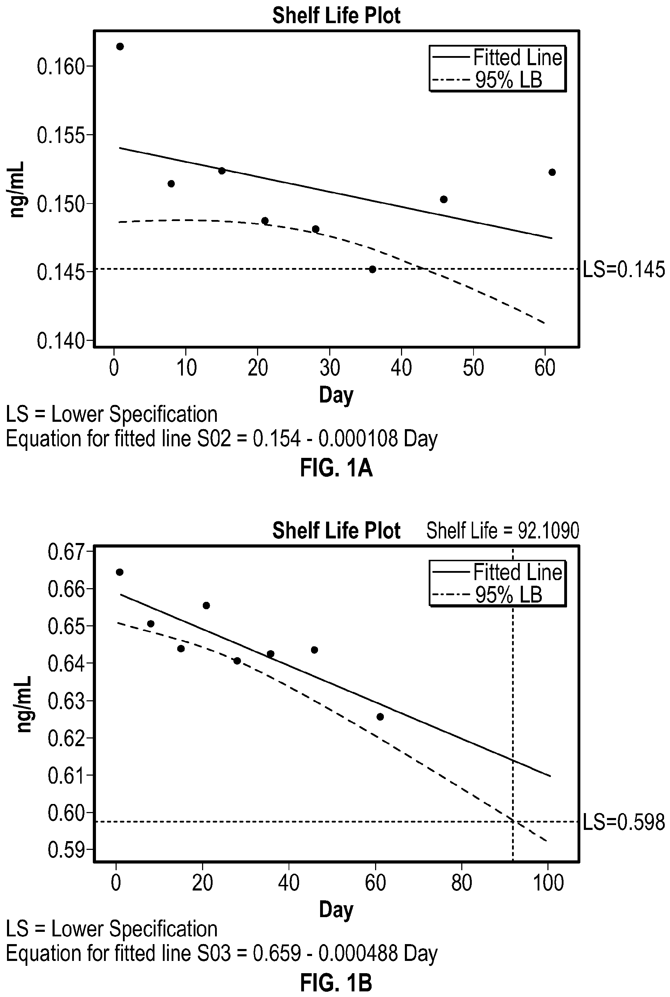

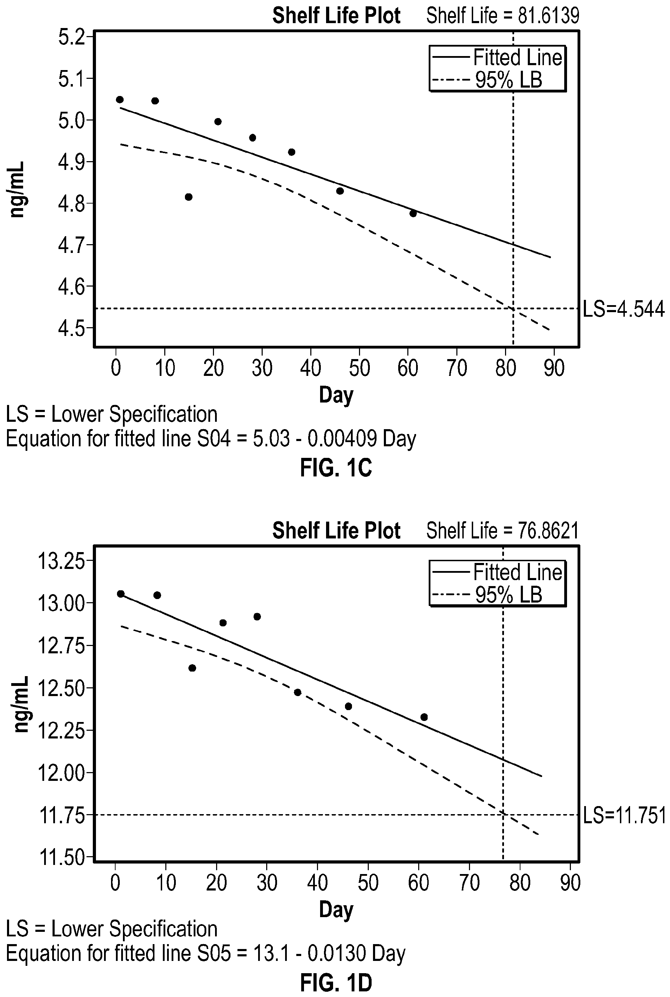

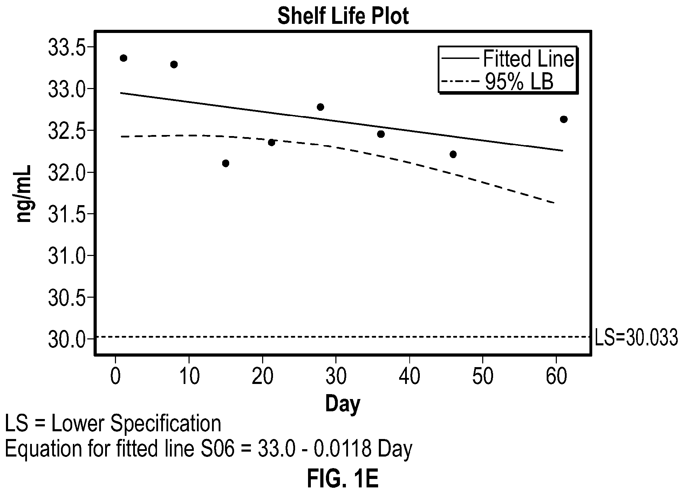

Referring now to FIGS. 1A-1E, shown therein are graphical plots showing the bioactivity and shelflife of MIS over a sixty (60)-day period for initial concentrations of MIS (at day 0) ranging from about 0.15 ng/mL to about 33.50 ng/mL in which the MIS is contained within an improved storage buffer constructed in accordance with the presently disclosed and/or claimed inventive concept(s) at a temperature ranging from about 2°C to about 8°C. As can be seen from these Figures, regardless of the initial concentration of the MIS, the MIS concentration of each sample at day 60 is reduced by only about 10% (or even less) when stored in the storage buffer. The at least one sugar alcohol (such as, by way of example, mannitol) component of the storage buffer stabilizes the MIS by preventing/mitigating the proteolysis cleavage that results in the activation of the MIS, thereby increasing both the shelf-life of the MIS, but also its bioactivity for use as a diagnostic reagent.

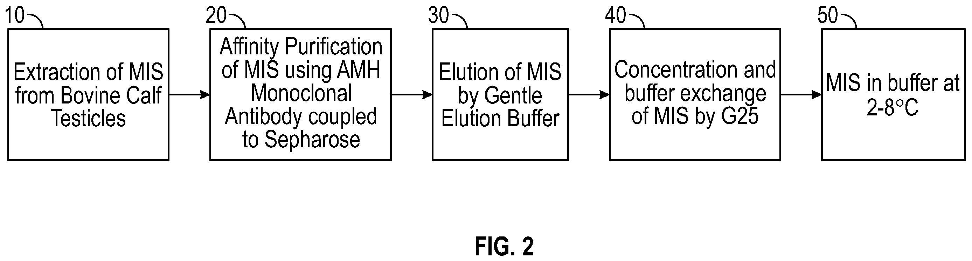

Referring now to FIG. 2, shown therein is a box diagram of a process for the extraction, purification, storage, and stabilization of purified MIS in a storage buffer constructed in accordance with the presently disclosed and/or claimed inventive concept(s). As shown in step 10, MIS is extracted (via methods commonly known in the art) from mammalian testicles, including, by way of example only, bovine calf testicles. Following extraction, the extracted MIS is purified (as shown in step 20) by any method commonly known in the art, including, without limitation, via affinity purification of the MIS via utilization of, for instance, MIS monoclonal antibody coupled to sepharose. Once the extracted MIS is bound to and associated with the particular purifying agent, the MIS is then eluted by at least one elution buffer, for instance, by way of example (and as shown in step 30), with pH neutral Gentle Elution Buffer. Following elution, the purified MIS is concentrated by methods commonly known in the art and the elution buffer is exchanged (as shown in step 40) with the storage buffer constructed in accordance with the presently disclosed and/or claimed inventive concept(s), the buffer exchange being accomplished via methods commonly known in the art, including, without limitation, sephadex G-25 methodologies. As shown in step 50, the purified and concentrated MIS is then stored within the storage buffer at a temperature ranging from about 2°C to about 8°C.

### NON-LIMITING EXAMPLES OF THE INVENTIVE CONCEPT(S)

Thus, in accordance with the presently disclosed and claimed inventive concept(s), there have been provided compositions and methods for stabilizing a Müllerian Inhibiting Substance. As described herein, the presently disclosed and claimed inventive concept(s) relate to embodiments of an improved Müllerian Inhibiting Substance storage buffer composition, as well as method(s) of stabilizing a Müllerian Inhibiting Substance within said improved buffer composition. Accordingly, the present disclosed and/or claimed inventive concept(s) fully satisfy the objectives and advantages set forth hereinabove. Although the presently disclosed and claimed inventive concept(s) has been described in conjunction with the specific drawings, experimentation, results, and language set forth hereinabove, it is evident that many alternatives, modifications, and variations will be apparent to those skilled in the art.

## Claims

1. A Müllerian Inhibiting Substance storage buffer composition, comprising:
deionized water;
at least one buffering agent;
at least one salt;
at least one sugar alcohol, wherein the at least one sugar alcohol is mannitol;
gelatin;
at least one surfactant;
protease-free bovine serum albumin; and
at least one biocide, wherein the Müllerian Inhibiting Substance storage buffer composition is suitable to be stored for use at a temperature range from about 2°C to about 8°C.

2. The storage buffer composition of claim 1, wherein the at least one buffering agent is selected from the group consisting of 4-(2-hydroxyethyl)-1-piperazineethanesulfonic acid buffer, [tris(hydroxymethyl)methylamino]propanesulfonic acid buffer, 2-(bis(2-hydroxyethyl)amino)acetic acid (bicine) buffer, tris(hydroxymethyl)aminomethane buffer, 3-[N-tris(hydroxymethyl)methylamino]-2-hydroxypropanesulfonic acid buffer, 2-[[1,3-dihydroxy-2-(hydroxymethyl)propan-2-yl]amino]ethanesulfonic acid buffer, 3-(N-morpholino)propanesulfonic acid buffer, piperazine-N-N'-bis(2-ethanesulfonic acid) buffer, dimethylarsenic acid, 2-(N-morpholino)ethanesulfonic acid buffer, N-cyclohexyl-2-aminoethanesulfonic acid buffer, citric acid buffer, acetic acid buffer, monopotassium phosphate buffer, borate buffer, and combinations thereof.

3. The storage buffer composition of claim 1, wherein the at least one buffering agent is 4-(2-hydroxyethyl)-1-piperazineethanesulfonic acid buffer, wherein preferably the 4-(2-hydroxyethyl)-1-piperazineethanesulfonic acid buffer comprises a concentration of about 50 mM.

4. The storage buffer composition of claim 1, wherein the at least one salt is sodium chloride, wherein the sodium chloride preferably comprises a concentration of about 150 mM.

5. The storage buffer composition of claim 1, wherein the gelatin is a liquid gelatin, wherein the liquid gelatin preferably comprises a concentration of about 6 grams/liter.

6. The storage buffer composition of claim 1, wherein the mannitol comprises a concentration of about 50 grams/liter.

7. The storage buffer composition of claim 1, wherein the at least one surfactant is selected from the group consisting of an anionic surfactant, a non-ionic surfactant, a cationic surfactant, an amphoteric surfactant, a zwitterionic surfactant, and combinations thereof.

8. The storage buffer composition of claim 1, wherein the at least one surfactant is polyethylene glycol sorbitan monolaurate, wherein the polyethylene glycol sorbitan monolaurate preferably comprises a concentration of about 1.1 grams/liter.

9. The storage buffer composition of claim 1, wherein the at least one biocide is selected from the group consisting of preservatives, antimicrobial agents, germicides, antibiotics, antibacterials, bactericides, antivirals, antifungals, antiprotozoals, antiparasites, anti-fouling agents, disinfectants, pesticides, fungicides, herbicides, insecticides, algicides, molluscicides, miticides, rodenticides, and combinations thereof.

10. The storage buffer composition of claim 1, wherein the at least one biocide is an ethanolic solution of brom-nitro-dioxane and methylisothiazolone.

11. The storage buffer composition of claim 10, wherein the ethanolic solution of brom-nitro-dioxane and methylisothiazolone comprises a concentration of about 2 milliliters/liter.

12. A method for stabilizing a purified Müllerian Inhibiting Substance within a Müllerian Inhibiting Substance storage buffer composition, the method comprising the steps of:
extracting a Müllerian Inhibiting Substance from a mammalian source to thereby form an extracted Müllerian Inhibiting Substance;
purifying the extracted Müllerian Inhibiting Substance to thereby form a purified Müllerian Inhibiting Substance; and
storing the purified Müllerian Inhibiting Substance in a storage buffer, the storage buffer comprising:
deionized water;
at least one buffering agent;
at least one salt;
at least one sugar alcohol, wherein the at least one sugar alcohol is mannitol;
gelatin;
at least one surfactant;
protease-free bovine serum albumin; and
at least one biocide,
wherein the storage buffer is maintained at a predetermined temperature range and stabilizes the purified Müllerian Inhibiting Substance contained therein, wherein the predetermined temperature range is from about 2°C to about 8°C.

## Patentansprüche

1. Lagerpufferzusammensetzung für Müller-hemmende Substanz, umfassend:
entionisiertes Wasser;
mindestens ein Puffermittel;
mindestens ein Salz;
mindestens einen Zuckeralkohol, wobei der mindestens eine Zuckeralkohol Mannitol ist;
Gelatine;
mindestens ein Tensid;
proteasefreies Rinderserumalbumin; und
mindestens ein Biozid, wobei die Lagerpufferzusammensetzung für Müller-hemmende Substanz geeignet ist, um zur Verwendung in einem Temperaturbereich von etwa 2 °C bis etwa 8 °C gelagert zu werden.

2. Lagerpufferzusammensetzung nach Anspruch 1, wobei das mindestens eine Puffermittel ausgewählt ist aus der Gruppe bestehend aus 4-(2-Hydroxyethyl)-1-piperazinethansulfonsäure-Puffer, [Tris(hydroxymethyl)methylamino]propansulfonsäure-Puffer, 2-(Bis(2-hydroxyethyl)amino)essigsäure-(Bicin)-Puffer, Tris(hydroxymethyl)aminomethan-Puffer, 3-[N-Tris(hydroxymethyl)methylamino]-2-hydroxypropansulfonsäure-Puffer, 2-[[1,3-Dihydroxy-2-(hydroxymethyl)propan-2-yl]amino]ethansulfonsäure-Puffer, 3-(N-Morpholino)propansulfonsäure-Puffer, Piperazin-N-N'-bis(2-ethansulfonsäure)-Puffer, Dimethylarsensäure, 2-(N-Morpholino)ethansulfonsäure-Puffer, N-Cyclohexyl-2-aminoethansulfonsäure-Puffer, Citronensäure-Puffer, Essigsäure-Puffer, Monokaliumphosphat-Puffer, Borat-Puffer und Kombinationen davon.

3. Lagerpufferzusammensetzung nach Anspruch 1, wobei das mindestens eine Puffermittel 4-(2-Hydroxyethyl)-1-piperazinethansulfonsäure-Puffer ist, wobei der 4-(2-Hydroxyethyl)-1-piperazinethansulfonsäure-Puffer vorzugsweise eine Konzentration von etwa 50 mM umfasst.

4. Lagerpufferzusammensetzung nach Anspruch 1, wobei das mindestens eine Salz Natriumchlorid ist, wobei das Natriumchlorid vorzugsweise eine Konzentration von etwa 150 mM umfasst.

5. Lagerpufferzusammensetzung nach Anspruch 1, wobei die Gelatine eine flüssige Gelatine ist, wobei die flüssige Gelatine vorzugsweise eine Konzentration von etwa 6 Gramm/Liter umfasst.

6. Lagerpufferzusammensetzung nach Anspruch 1, wobei das Mannitol eine Konzentration von etwa 50 Gramm/Liter umfasst.

7. Lagerpufferzusammensetzung nach Anspruch 1, wobei das mindestens eine Tensid ausgewählt ist aus der Gruppe bestehend aus einem anionischen Tensid, einem nichtionischen Tensid, einem kationischen Tensid, einem amphoteren Tensid, einem zwitterionischen Tensid, und Kombinationen davon.

8. Lagerpufferzusammensetzung nach Anspruch 1, wobei das mindestens eine Tensid Polyethylenglykolsorbitanmonolaurat ist, wobei das Polyethylenglykolsorbitanmonolaurat vorzugsweise eine Konzentration von etwa 1,1 Gramm/Liter umfasst.

9. Lagerpufferzusammensetzung nach Anspruch 1, wobei das mindestens eine Biozid ausgewählt ist aus der Gruppe bestehend aus Konservierungsmitteln, antimikrobiellen Mitteln, Germiziden, Antibiotika, antibakteriellen Mitteln, Bakteriziden, antiviralen Mitteln, Antimykotika, Antiprotozotika, Antiparasitika, Antifouling-Mitteln, Desinfektionsmitteln, Pestiziden, Fungiziden, Herbiziden, Insektiziden, Algiziden, Molluskiziden, Mitiziden, Rodentiziden und Kombinationen davon.

10. Lagerpufferzusammensetzung nach Anspruch 1, wobei das mindestens eine Biozid eine ethanolische Lösung von Bromnitrodioxan und Methylisothiazolon ist.

11. Lagerpufferzusammensetzung nach Anspruch 10, wobei die ethanolische Lösung von Bromnitrodioxan und Methylisothiazolon eine Konzentration von etwa 2 Milliliter/Liter umfasst.

12. Verfahren zum Stabilisieren einer gereinigten Müller-hemmenden Substanz innerhalb einer Lagerpufferzusammensetzung für Müller-hemmende Substanz, wobei das Verfahren die folgenden Schritte umfasst:
Extrahieren einer Müller-hemmenden Substanz aus einer Säugerquelle, um dadurch eine extrahierte Müller-hemmenden Substanz zu bilden;
Reinigen der extrahierten Müller-hemmenden Substanz, um dadurch eine gereinigte Müller-hemmende Substanz zu bilden; und
Lagern der gereinigten Müller-hemmenden Substanz in einem Lagerpuffer, wobei der Lagerpuffer umfasst:
entionisiertes Wasser;
mindestens ein Puffermittel;
mindestens ein Salz;
mindestens einen Zuckeralkohol, wobei der mindestens eine Zuckeralkohol Mannitol ist;
Gelatine;
mindestens ein Tensid;
proteasefreies Rinderserumalbumin; und
mindestens ein Biozid,
wobei der Lagerpuffer auf einem vorbestimmten Temperaturbereich gehalten wird und die darin enthaltene gereinigte Müller-hemmende Substanz stabilisiert, wobei der vorbestimmte Temperaturbereich von etwa 2 °C bis etwa 8 °C beträgt.

## Revendications

1. Composition tampon de stockage de substance inhibitrice Müllérienne, comprenant :
de l'eau désionisée ;
au moins un agent tampon ;
au moins un sel ;
au moins un alcool de sucre, dans lequel au moins un alcool de sucre est le mannitol ;
de la gélatine ;
au moins un agent tensioactif ;
de l'albumine sérique bovine exempte de protéase ; et
au moins un biocide, dans lequel la composition tampon de stockage de substance inhibitrice Müllérienne pouvant être stockée en vue d'une utilisation à une température comprise entre 2°C et 8°C environ.

2. Composition tampon de stockage de la revendication 1, dans laquelle ledit au moins un agent tampon est choisi dans le groupe constitué du tampon d'acide 4-(2-hydroxyéthyl)-1-pipérazineéthanesulfonique, le tampon d'acide [tris(hydroxyméthyl)méthylamino]propanesulfonique, le tampon d'acide 2-(bis(2-hydroxyéthyl)amino)acétique (bicine), le tampon tris(hydroxyméthyl)aminométhane, le tampon d'acide 3-[N-tris(hydroxyméthyl)méthylamino]-2-hydroxypropanesulfonique, le tampon d'acide 2-[[1,3-dihydroxy-2-(hydroxyméthyl)propan-2-yl]amino]éthanesulfonique, le tampon d'acide 3-(N-morpholino)propanesulfonique, le tampon d'acide pipérazine-N-N'-bis(2-éthanesulfonique), l'acide diméthylarsénique, le tampon d'acide 2-(N-morpholino)éthanesulfonique, le tampon d'acide N-cyclohexyl-2-aminoéthanesulfonique, le tampon d'acide citrique, le tampon d'acide acétique, le tampon phosphate monopotassique, le tampon borate, et leurs combinaisons.

3. Composition tampon de stockage de la revendication 1, dans laquelle ledit au moins un agent tampon est un tampon d'acide 4-(2-hydroxyéthyl)-1-pipérazineéthanesulfonique, dans lequel le tampon d'acide 4-(2-hydroxyéthyl)-1-pipérazineéthanesulfonique comprend de préférence une concentration d'environ 50 mM.

4. Composition tampon de stockage de la revendication 1, dans laquelle ledit au moins un sel est le chlorure de sodium, dans laquelle le chlorure de sodium comprend de préférence une concentration d'environ 150 mM.

5. Composition tampon de stockage de la revendication 1, dans laquelle la gélatine est une gélatine liquide, dans laquelle la gélatine liquide comprend de préférence une concentration d'environ 6 grammes/litre.

6. Composition tampon de stockage de la revendication 1, dans laquelle le mannitol comprend une concentration d'environ 50 grammes/litre.

7. Composition tampon de stockage de la revendication 1, dans laquelle ledit au moins un agent tensioactif est choisi dans le groupe constitué d'un agent tensioactif anionique, d'un agent tensioactif non ionique, d'un agent tensioactif cationique, d'un agent tensioactif amphotère, d'un agent tensioactif zwitterionique, et de leurs combinaisons.

8. Composition tampon de stockage de la revendication 1, dans laquelle ledit au moins un agent tensioactif est le monolaurate de polyéthylène glycol sorbitane, dans laquelle le monolaurate de polyéthylène glycol sorbitane comprend de préférence une concentration d'environ 1,1 gramme/litre.

9. Composition tampon de stockage de la revendication 1, dans laquelle ladite au moins un biocide est choisi dans le groupe constitué des conservateurs, les agents antimicrobiens, les germicides, les antibiotiques, les antibactériens, les bactéricides, les antiviraux, les antifongiques, les antiprotozoaires, les antiparasites, les agents antisalissures, les désinfectants, les pesticides, les fongicides, les herbicides, les insecticides, les algicides, les molluscicides, les acaricides, les rodenticides, et les combinaisons de ceux-ci.

10. Composition tampon de stockage de la revendication 1, dans laquelle ledit au moins un biocide est une solution éthanolique de brom-nitro-dioxane et de méthylisothiazolone.

11. Composition tampon de stockage de la revendication 10, dans laquelle la solution éthanolique de brom-nitro-dioxane et de méthylisothiazolone comprend une concentration d'environ 2 millilitres/litre.

12. Procédé de stabilisation d'une substance inhibitrice Müllérienne purifiée dans une composition tampon de stockage de substance inhibitrice Müllérienne, comprenant les étapes consistant à :
extraire une substance inhibitrice Müllérienne à partir d'une source mammifère pour obtenir une substance inhibitrice Müllérienne extraite ;
purifier la substance inhibitrice Müllérienne extraite pour obtenir une substance inhibitrice Müllérienne purifiée ; et
stocker la substance inhibitrice Müllérienne purifiée dans un tampon de stockage, le tampon de stockage comprenant ;
de l'eau désionisée ;
au moins un agent tampon ;
au moins un sel ;
au moins un alcool de sucre, dans lequel ledit au moins un alcool de sucre est le mannitol ;
la gélatine ;
au moins un agent tensioactif ;
de l'albumine sérique bovine exempte de protéase ; et
au moins un biocide,
dans lequel le tampon de stockage est maintenu à une température prédéterminée et stabilise la substance inhibitrice Müllérienne purifiée qu'il contient, la température prédéterminée étant comprise entre environ 2°C et environ 8°C.
